(19) Europäisches Patentamt
European Patent Office
Office européen des brevets

(11) Veröffentlichungsnummer: **0 232 284**
**B1**

(12) **EUROPÄISCHE PATENTSCHRIFT**

(45) Veröffentlichungstag der Patentschrift: **28.11.90**

(51) Int. Cl.$^5$: **C 07 K 7/26,** A 61 K 37/02

(21) Anmeldenummer: **86903256.5**

(22) Anmeldetag: **20.06.86**

(86) Internationale Anmeldenummer:
**PCT/DE86/00259**

(87) Internationale Veröffentlichungsnummer:
**WO 87/00181 15.01.87 Gazette 87/01**

(54) **NEUE SOMATOSTATIN-DERIVATE.**

(30) Priorität: **25.06.85 DE 3522638**

(43) Veröffentlichungstag der Anmeldung:
**19.08.87 Patentblatt 87/34**

(45) Bekanntmachung des Hinweises auf die Patenterteilung:
**28.11.90 Patentblatt 90/48**

(84) Benannte Vertragsstaaten:
**AT BE CH DE FR GB IT LI LU NL SE**

(56) Entgegenhaltungen:
**EP-A-0 000 330**
**EP-A-0 114 787**
**US-A-4 061 608**

**Chemical Abstracts, Band 101, 1984, (Columbus, Ohio, US), W G Bessler et al.: "Synthetic peptide derivatives of bacterial lipoprotein and their interaction with lymphocyte plasma membranes", Seite 701, Zusamenfassung Nr. 211690s.**

(73) Patentinhaber: **DIAMALT AKTIENGESELLSCHAFT**
**Georg-Reismüller-Strasse 34**
**D-8000 München 50 (DE)**

(72) Erfinder: **JUNG, Günter**
**Auf der Morgenstelle 18**
**D-7400 Tübingen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 232 284 B1

**Beschreibung**

Die Erfindung betrifft Somatostatin-Derivate der allgemeinen Formel I

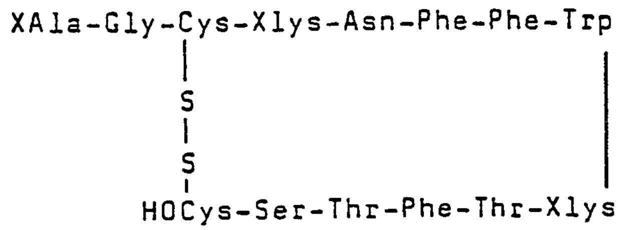

(I),

worin mindestens einer der Reste X einen an die freie Aminogruppe des Alanins oder Lysins gebundenen Rest der Formel II

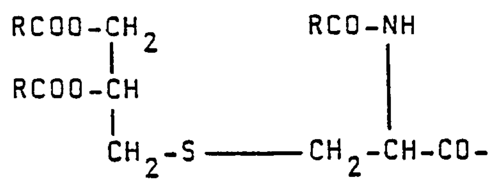

(II),

mit R in der Bedeutung von 7 bis 23 Kohlenstoffatome enthaltenden Alkylgruppen gleichen oder verschiedenen Kettenlänge darstellt und die gegebenenfalls verbleibenden Reste X Wasserstoffatome bedeuten.

Ferner betrifft die Erfindung ein Verfahren zur Herstellung dieser Somatosatin-Derivate und pharmazeutische Präparate, die diese Verbindungen enthalten.

Somatostatin ist bekanntlich eine pharmakologisch wirksame Substanz die unter anderen zur Behandlung von Diabetis mellitus und gastrointestinaler Erkrankungen Anwendung findet S. M. McCann Ann. Rev. Pharmacol. Toxicol 1982, *22*, 491—515, K. Gerbitz Nachr. Chem. Techn. 1975, *23*, 355—357, K. Gyr Trends in Pharmacol. Sci., 1983, *3*, 367—369 und S. Reichlin N. Engl. Med. 1983, *309*, 1495—1501 und 1556—1563).

Nachteile dieser Verbindung sind unter anderem ihre nicht ausreichend spezifische Wirksamkeit und ihr rascher Wirkungsabfall.

Die erfindungsgemäßen Somatostatin-Derivate der allgemeinen Formel I besitzen die gleiche Wirkungsrichtung wie Somatostatin selbst, zeichnen sich aber gegenüber dieser Verbindung durch eine speizifischere Wirksamkeit und eine längere Wirkungsdauer aus.

Die erfindungsgemäßen Somatostatin-Derivate können aus Somatostatin und den Säuren der allgemeinen Formel III

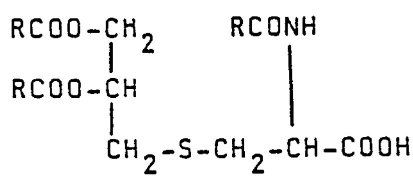

(III),

worin R die im Anspruch 1 genannte Bedeutung besitzt, oder einem reaktionsfähigem Derivat derselben unter Bedingungen hergestellt werden, welche man in der Peptidsynthese üblicherweise zur Herstellung von Säureamid-Bindungen verwendet (Houben-Weyl, Methoden der organischen Chemie, Georg Thieme Verlag Stuttgart BRD, 4. Auflage, Band XV/1 Synthese von Peptiden, Teil 1, 1974, S. 28 ff).

So kann man beispielsweise die Carbonsäuren unter den bekannten Bedingungen mit Somatostatin in Gegenwart von Dicyclohexylcarbodiimid umsetzen oder man überführt die Säuren in die entsprechenden Säurechloride oder gemischten Anhydride und setzt diese in an sich bekannte Weise mit Somatostatin um.

Die als Ausgangsverbindungen verwendeten Säuren der allgemeinen Formel III können als Substituenten R 7 bis 23 Kohlenstoffatome enthaltende Alkylgruppen gleicher oder verschiedener Kettenlänge haben. Diese Alkylgruppen sind vorzugsweise geradkettig und besitzen eine ungerade Anzahl von Kohlenstoffatomen. Als Alkylgruppen seien beispielsweise genannt: Die Heptylgruppe, die Nonylgruppe, die Undecylgruppe, die Tridecylgruppe, die Pentadecylgruppe, die Heptadecylgruppe, die Nonadecylgruppe, die Heneicosylgruppe und die Tricosylgruppe.

Als Ausgangsverbindungen für das erfindungsgemäße Verfahren eignen sich beispielsweise folgende Säuren das N - Palmitoyl - S - (2[R,S],3 - dilanryloxy - propyl) - L - cystein, das N - Palmitoyl - S -

(2[R,S],3 - dipalmitoyloxy - propyl) - L - cystein, das N - Octanoyl - S - (2[R,S],3 - didecanoyloxy - propyl) - L - cystein, das N - Octanoyl - S - (2[R,S],3 - didodecanoyloxy - propyl) - L - cystein, das N - Octanoyl - S - (2[R,S],3 - dioctanoyloxy - propyl) - L - cystein, das N - Eicosanoyl - S - (2[R,S],3 - dioctanoyloxy - propyl) - L - cystein, das N - Tetracosanoyl - S - (2[R,S],3 - dioctanoyloxy - propyl) - L - cystein und das N - Stearoyl - S - (2[R,S],3 - distearoyloxy - propyl) - L - cystein.

Die Säuren der allgemeinen Formel III können durch Acylierung von Verbindungen der Formel IV

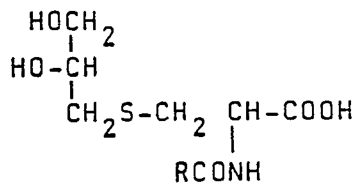

(IV),

hergestellt werden, welche ihrerseits nach dem in der Europäischen Patentanmeldung 114787 beschriebenen Verfahren synthetisiert werden können.

Die erfindungsgemäßen Verbindungen können durch Zusatz der üblichen Hilfsmittel und/oder Trägersubstanzen zu pharmazeutischen Präparaten verarbeitet werden. So kann mann beispielsweise die stärker hydrophilen Somatostatin-Derivate der allgemeinen Formel I gegebenenfalls unter Zusatz von Lösungsvermittlern (etwa bis zu 1 Gewichtsprozent Cremophor® oder bis zu 30 Gewichtsprozent Propylenglycol, Puffersubstanzen (Phosphatpuffer, Tris-Puffer) Kochsalz etc. in wässrige Injektionslösungen oder Infusionslösungen überführen. Andererseits kann man sie auch unter Zusatz der üblichen Träger (Lactose, Galactose, niedermolekulares Polyvinylpyrrolidon, etc.) zu Trockensubstanzen verarbeiten, die vor der Injektion oder Infusion mit bidestilliertem Wasser versetzt werden. Die lipophilen Somatostatin-Derivate der allgemeinen Formel I lassen sich mittels Ölen (wie Sojaöl) und den üblichen Emulgatoren (wie bis zu 10 Gewichtsprozent Lecitin oder einem Phospholipid) in zur Injektion oder Infusion geeignete Ölemulsionen überführen. Durch die üblichen Vorkehrungen ist selbstverständlich zu gewährleisten, daß die hergestellten Präparate, die pro Dosis üblicherweise 100 µg bis 5 mg Wirkstoff enthalten, keimfrei sind.

Die erfindungsgemäßen Präparate können beispielsweise zur Behandlung der Diabetes mellitus oder zur Behandlung gastrointestinaler Erkrankungen verwendet werden. Die üblicherweise anzuwendende Tagesdosis beträgt 50 µg bis 200 µg Somatostatin-Derivat/kg Körpergewicht.

Das nachfolgende Ausführungsbeispiel dient zur Erläuterung des erfindungsgemäßen Verfahrens.

Beispiel

200 mg N - Palmitoyl - S - (2[R,S],3 - dipalmitoyl - propyl) - L - cystein werden in 5 ml eines Gemischs aus 2 Volumteilen Dimethylformamid und 1 Volumteil Chloroform mit 30 mg Butanol und 45 mg Dicyclohexylcarbodiimid versetzt und 30 Minuten lang bei 0°C gerührt. Dann setzt man der Reaktionsmischung 90 mg Somatostatin und 16 mg N-Methylmorpholin in 5 ml Dimethylformamid-Chloroform (Mischungsverhältnis wie beschrieben) zu und rührt 6 Stunden lang bei Raumtemperatur.

Man engt die Reaktionsmischung im Vakuum ein reinigt den Rückstand mittels Gelchromatographie (Sephadex® LH 20, Chloroform-Methanol 1 + 1) und erhält 40 mg Tri - N - [N - Palmitoyl - S - (2[R,S],3 - dipalmitoyl - propyl) - L - cysteinyl]-somatostatin.

**Patentansprüche**

1. Somatostatin-Derivate der allgemeinen Formel I

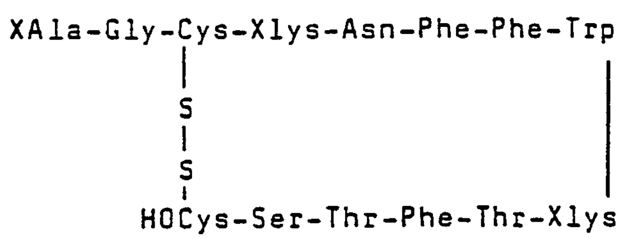

(I),

worin mindestens einer der Reste X einen an die freie Aminogruppe des Alanins oder Lysins gebundenen Rest der Formel II

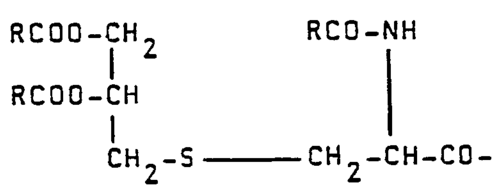

(II),

mit R in der Bedeutung von 7 bis 23 Kohlenstoffatome enthaltenden Alkylgruppen gleichen oder verschiedenen Kettenlänge darstellt und die gegebenenfalls verbleibenden Reste X Wasserstoffatome bedeuten.

2. Somatostatin-Derivate der allgemeinen Formel I gemäß Patentanspruch 1, dadurch gekennzeichnet, daß die Gruppe R jeweils einen n-Pentadecylrest oder einen n-Heptadecylrest darstellen.

3. Somatostatin-Derivate der allgemeinen Formel I gemäß Patentanspruch 1 und 2, dadurch gekennzeichnet, daß alle Reste X von Wasserstoff verschieden sind.

4. Verfahren zur Herstellung von Somatostatin-Derivaten der allgemeinen Formel I gemäß Patentanspruch 1, dadurch gekennzeichnet, daß man Somatostatin mit einer Säure der allgemeinen Formel III

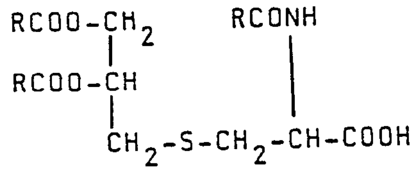

(III),

worin R die im Anspruch 1 genannte Bedeutung besitzt, oder einem reaktionsfähigem Derivat derselben N-Acyliert.

5. Pharmazeutische Präparate, gekennzeichnet durch einen Gehalt an einem Somatostatin-Derivat gemäß Anspruch 1 bis 3.

**Revendications**

1. Dérivés de la somatostatine, de formule générale

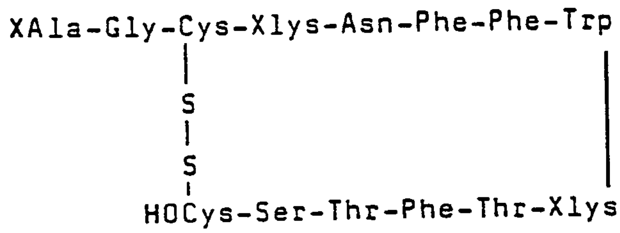

(I),

dans laquelle au moins l'un des restes X représente un reste (relié au groupe amino libre de l'alanine ou de la lysine) de formule II

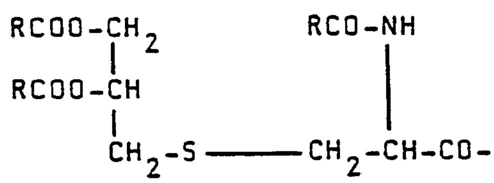

(II),

dans laquelle R représente des groupes alkyles, de même longueur de chaîne ou de longueur de chaîne différentes, contenant 7 à 23 atomes de carbone, et les restes X éventuellement restants sont des atomes d'hydrogène.

2. Dérivés de la somatostatine de formule générale I selon la revendication 1, caractérisés en ce que les groupes R représentent à chaque fois un reste pentadécyle ou un reste heptadécyle.

3. Dérivés de la somatostatine de formule générale I selon la revendication 1 et 2, caractérisés en ce que tous les restes X sont des restes différents d'un atome d'hydrogène.

4. Procédé pour préparer des dérivés de la somatostatine de formule générale I selon la revendication 1, procédé caractérisé en ce qu'on soumet la somatostatine à une acylation de l'azote à l'aide d'un acide de formule générale III

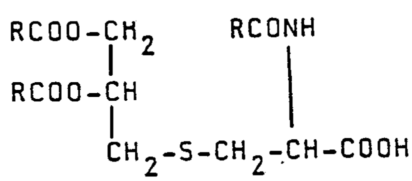

(III),

dans laquelle R a le sens indiqué à la revendication 1, ou à l'aide d'un dérivé réactif de cet acide.

5. Préparations pharmaceutique, caractérisées en ce qu'elles contiennent un dérivé de la somatostatine selon l'une des revendications 1 à 3.

**Claims**

1. Somatostatin derivatives of the general formula I

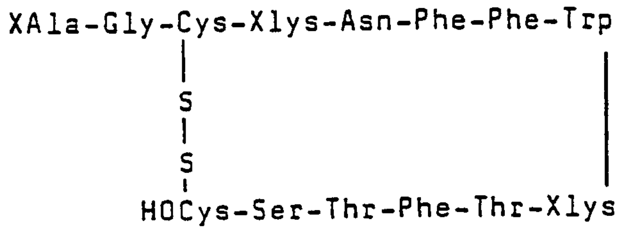

(I),

in which at least one of the radicals X represents a radical of the formula II

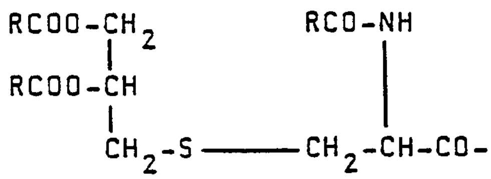

(II),

bonded to the free amino group of alanine or lysine, in which R represents alkyl groups of identical or different chain lengths and containing from 7 to 23 carbon atoms, and any radical X that may remain represents a hydrogen atom.

2. Somatostatin derivatives of the general formula I according to patent claim 1, characterised in that each of the groups R represents an n-pentadecyl radical or an n-heptadecyl radical.

3. Somatostatin derivatives of the general formula I according to patent claim 1 and 2, characterised in that all of the radicals X have a meaning other than hydrogen.

4. Process for the preparation of somatostatin derivatives of the general formula I according to patent claim 1, characterised in that somatostatin is N-acylated with an acid of the general formula III

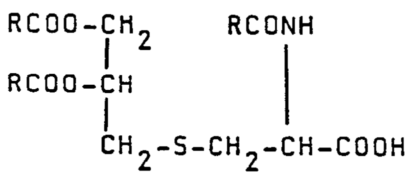

(III),

in which R has the meaning given in claim 1, or with a reactive derivative thereof.

5. Pharmaceutical preparations, characterised in that they contain a somatostatin derivative according to claims 1 to 3.